# EUROPEAN PATENT APPLICATION

(11) **EP 3 257 434 A1**
(43) Date of publication of application: **20.12.2017**
(21) Application number: 16175102.9
(22) Date of filing: 17.06.2016
(51) Int. Cl.: A61B 3/04, G02C 7/08

(54) **SUBJECTIVE REFRACTION APPARATUS**

(71) Applicant: ESSILOR INTERNATIONAL (Compagnie Générale d'Optique), 94220 Charenton-le-Pont (FR); Adaptive EyeWorks BVBA, 2000 Antwerp (BE)
(72) Inventor: KOH, Patricia, 339338 Singapore (SG); KHALIFA, Aly, Raleigh, NC 27604 (US); FRIEDMAN, John, 1970 Wezembeek-Oppem (BE); VERGARA SANZ, Jimena, Bogota (CO); VAN ASBECK, Frederik, 5213AR 's-Hertogenbosch (NL)
(74) Representative: Jacobacci Coralis Harle

(57) **Abstract**

A subjective refraction apparatus provides a variable optical correction along an optical axis. The subjective refraction apparatus comprises a pair of lenses (70, 80; 50, 60) mounted on a mount (20; 30) in such a manner that the lenses (70, 80; 50, 60) are movable with respect to each other in a direction perpendicular to the optical axis for generating a variable spherical power along the optical axis.

The mount (20; 30) is provided with a support (24; 34) defining a space for removably receiving an additional lens on the optical axis.

## Description

### TECHNICAL FIELD OF THE INVENTION

The invention relates to the correction of ametropia.

More precisely the invention relates to a subjective refraction apparatus.

### BACKGROUND INFORMATION AND PRIOR ART

The necessary correction for compensating an individual's ametropia is generally determined by an optometrist or an ophthalmologist using a test known as *"subjective refraction",* during which the individual looks through a refraction apparatus adapted to generate a variable correction.

Such a refraction apparatus may be a phoropter or trial frames, for example.

The subjective refraction makes it possible to determine a refraction value (such as a spherical refractive power, a cylindrical refractive power or a cylinder axis) that an ophthalmic lens placed before the individual's eye should provide in order to compensate for the individual's ametropia.

Currently, subjective refraction tests meant to determine spherical and cylinder powers are performed using a trial lens set or a phoropter. A trial lens set is big and bulky, while a phoropter is expensive and space consuming. For a mobile eye care practitioner, it is inconvenient to bring the large set of trial lenses around. This is especially so in rural area whereby transportation is done through small motor-bikes or bicycles.

There is a method known as Alvarez concept (as described for instance in document US3507565A) whereby it allows combination of spherical prescription within a frame through displacement of 2 adjacent lenses. It is originally meant for end-users whereby they can adjust the power themselves at whatever distance they looking at. However, often the wearers may tend to over-correct themselves because there is no guideline on the distance target. Cylinder prescription is also possible using the same concept but it is more costly to manufacture and the measurement process is complicated. Most of the Alvarez concept tools in the market only consist of spherical measurement with a limitation in the range of power.

Therefore it is important to have a cheap, easy to use, small, compact set of tools that can be used to perform subjective refraction on both spherical power and cylinder power.

### SUMMARY OF THE INVENTION

In this context, the invention provides a subjective refraction apparatus providing a variable optical correction along an optical axis and comprising a pair of lenses mounted on a mount in such a manner that the lenses are movable with respect to each other in a direction perpendicular to the optical axis for generating a variable spherical power along the optical axis, characterized in that the mount is provided with a support defining a space for removably receiving an additional lens on the optical axis.

Such a subjective refraction apparatus makes it possible to test spherical correction by adjusting the variable spherical power and possibly to test cylindrical correction by introducing an additional lens providing cylindrical power, while avoiding a complex construction that would make the apparatus cumbersome and expensive.

According to possible additional features (which are to be understood as non limiting):
- the subjective refraction apparatus further comprises said additional lens mounted onto said support;
- said additional lens has a cylindrical power along the optical axis;
- said additional lens has a spherical power along the optical axis;
- the mount includes markings along a periphery of said space;
- the mount extends perpendicularly to the optical axis;
- said support extends on a side of said mount opposite another side of said mount facing one of said movable lenses;
- said support extends over less than half the periphery of the space for receiving additional lens;
- a spherical power scale is affixed to a first movable lens among the movable lenses;
- an index pointing on the spherical power scale is affixed to the second movable lens among the movable lenses;
- said spherical power scale extends on a side of the first movable lens directed towards the space for receiving the additional lens;
- said mount defines a window in correspondence with said spherical power scale;
- each movable lens has a threaded part engaging a screw;
- said screw is rotatable in the mount in such a manner that the movable lenses move symmetrically (in opposite directions) with respect to the mount;
- said threaded part is a transparent part integral with an optical part of the concerned movable lens;
- the mount is mounted on a frame at an adjustable position perpendicularly to the optical axis;
- a pinion attached to a button engages a rack affixed to the mount;
- the subjective refraction apparatus further includes a pair of temples;
- each temple is mounted to the frame.

Other features and advantages of the embodiments of the present invention will be better understood upon reading of preferred embodiments thereof with reference to the appended drawings.

### BRIEF DESCRIPTION OF THE DRAWINGS

Figure 1 shows a possible example of a subjective refraction apparatus according to the invention.
Figure 2 is an exploded view of the subjective refraction apparatus of Figure 1.
Figure 3 is a detailed section showing a rack and pinion mechanism used in the subjective refraction apparatus of Figure 1.
Figure 4 is a detailed section showing a spherical power adjustment mechanism in the subjective refraction apparatus of Figure 1.
Figure 5 shows a pair of lenses used in the subjective refraction apparatus of Figure 1, in a first relative position.
Figure 6 shows the pair of lenses of Figure 5, in a second relative position.
Figure 7 shows a possible variation for a mount of the subjective refraction apparatus of Figure 1.

### DETAILED DESCRIPTION OF EXAMPLE(S)

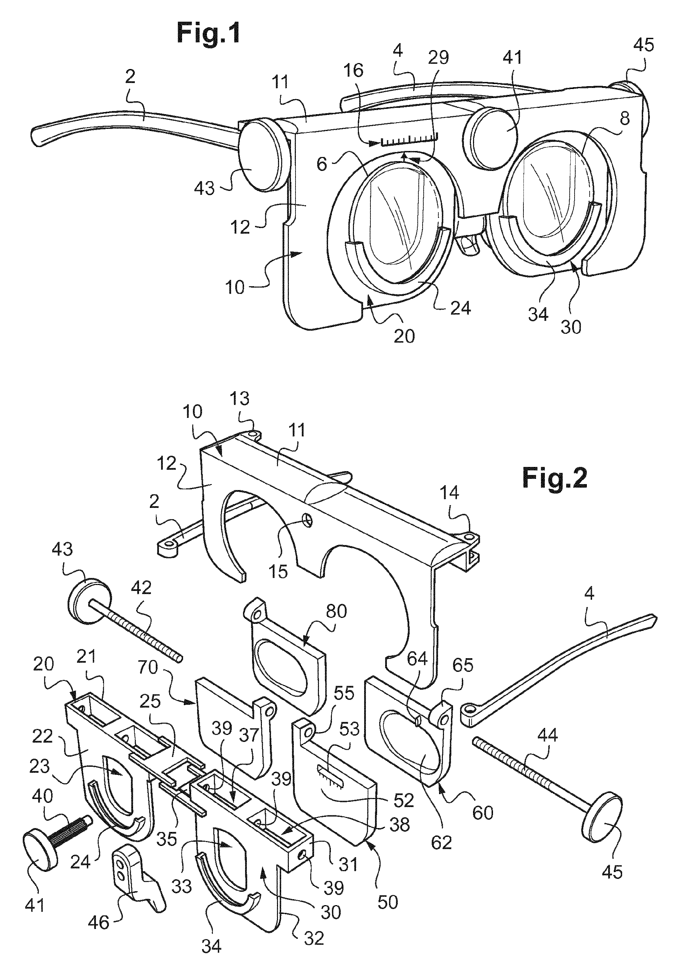

Figures 1 and 2 show a possible embodiment of a subjective refraction apparatus according to the invention.

This subjective refraction apparatus comprises a frame 10, a right temple 2, a left temple 4, a right lens mount 20 and a left lens mount 30.

The frame 10 and/or the right temple 2 and/or the left temple 4 and/or the right lens mount 20 and/or the left lens mount 30 are for instance made of a plastic material, here a translucent (and preferably strong and/or polymeric) plastic material, such as polycarbonate, polyamide, or acrylonitrile styrene butadiene (ABS). In other embodiments, these parts could however be opaque and/or be made of another material than plastics.

The frame 10 has a cover part 11 covering the right lens mount 20 and the left lens mount 30 when the subjective refraction apparatus is assembled.

The frame 10 also comprises a front plate 12 extending vertically (downward) from a front edge of the cover part 11.

It may be noted here that the present description is made considering the subjective refraction apparatus is mountable on an individual's head (thanks in particular to the above mentioned right temple 2 and left temple 4). Hence, directions are to be understood when mounted on the individual's head. In particular, "front" refers to portions situated opposite the individual's head, while "back" or "rear" refer to portions facing the individual's head.

The front plate 12 extends only partially in the concerned vertical plane not to block light impinging on the lenses, as described below.

The frame 10 lastly comprises hinge elements 13, 14 extending from the rear end of the cover part 11, at each lateral end thereof, in order to hingedly mount the right temple 2 and the left temple 4, respectively, to the frame 10.

In addition, a nosepad 46 is mounted to the rear face of the front plate 12 of the frame 10.

The right lens mount 20 and the left lens mount 30 each comprise a sliding part 21, 31 arranged in the frame 10 (just underneath the cover 11) so as to be laterally movable, for instance by engaging a corresponding guide (not shown) formed in the frame 10.

Further, the right lens mount 20 and the left lens mount 30 each comprise a plate 22, 32 extending vertically (downward) from (a front portion of) the sliding part 21, 31 of the concerned lens mount 20, 30.

Each plate 22, 32 has a central opening 23, 33 for light impinging on the lenses and a support 24, 34 extending in the bottom region of the concerned plate 22, 32 and partially surrounding the corresponding central opening 23, 33.

Each support 24, 34 extends on the front side of the concerned plate 22, 32 while the lenses 50, 60, 70, 80 (further described below) are mounted facing the rear side of the concerned plate 22, 32.

Each support 24, 34 is designed to hold an additional lens 6, 8, as shown in Figure 1. Each additional lens 6, 8 is here a lens having a cylindrical power along the optical axis defined by the lenses 50, 60, 70, 80 mounted on the corresponding mount 20, 30 (see below).

Each support 24, 34 is further designed to allow the concerned additional lens 6, 8 to rotate about the optical axis such that an axis of the cylindrical correction provided by the concerned additional lens 6, 8 may be adjusted as desired by the optometrist during the subjective refraction test.

In the present case, each support 24, 34 comprises a cylindrical base partially surrounding the central opening 23, 33 (in a bottom part of the plate 22, 32) and a retaining portion extending (upward) from the cylindrical base to define a slot for receiving an additional lens. An additional lens 6, 8 (of circular shape) can thus rest on the cylindrical base and rotate in the support 24, 34 (thanks to the circular shape of the cylindrical base) while being held in the support thanks to the retaining portion.

According to a possible variation, the additional lens mounted on support 24 (and/or the additional lens mounted on support 34) may be a lens having a spherical power along the optical axis. This may be used for instance to obtain a very high spherical power value by combination of such an additional lens and the corresponding lens assembly 70, 80 (or 50, 60), when such a very high spherical power value is not obtainable by use of the lens assembly 70, 80 (or 50, 60) itself.

The right lens mount 20 and the left lens mount 30 further each comprise an arm 25, 35 extending in a substantially horizontal plane from an inner lateral wall of the concerned sliding part 21, 31 *(i.e.* a lateral wall of the concerned sliding part 21, 31 situated in the middle portion of the frame 10 when the subjective refraction apparatus is assembled).

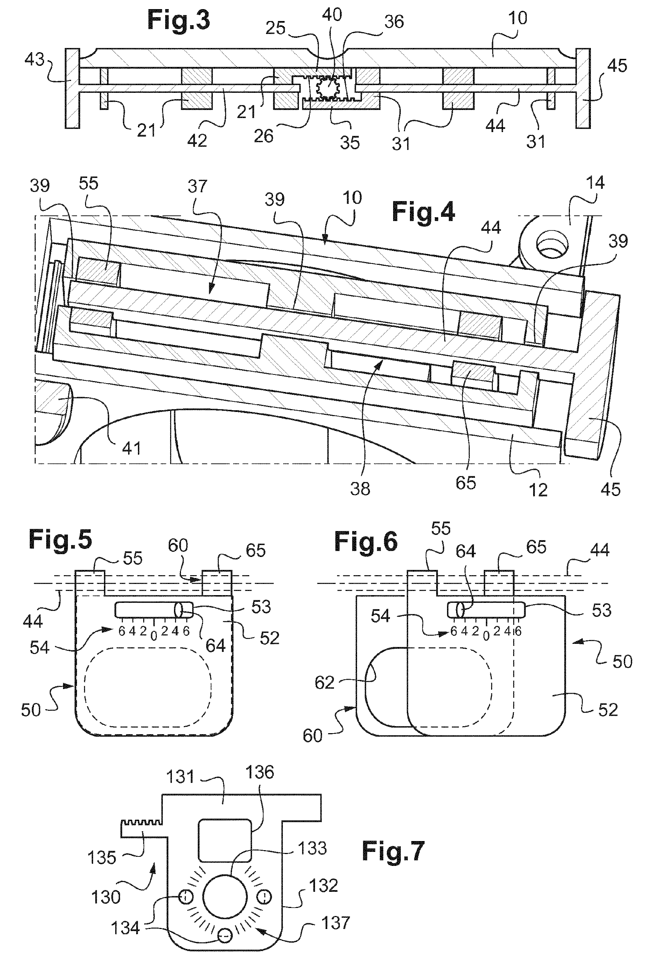

As clearly visible on Figures 2 and 3, in a mounted position, the arm 25 of the right lens mount 20 is however situated above the arm 35 of the left lens mount 30; in addition, the arm 25 of the right lens mount 20 and the arm 35 of the left lens mount 30 are separated by a space accommodating a pinion 40.

The pinion 40 is mounted in a hole 15 provided in (the central part) of the front plate 12 of the frame 10 and is affixed to a first button 41.

Each arm 25, 35 has teeth 26, 36 on its face facing the pinion 40 (see Figure 3) and thus forms a rack which engages with the pinion 40.

In view of the above construction, when the user (e.g. an optometrist) rotates the first button 41, both sliding parts 21, 31 (and thus both the right lens mount 20 and the left lens mount 30) are simultaneously moved, but in opposite directions, which makes it possible to adjust the pupillary distance (PD), for instance from a first value between 55 mm and 60 mm to a second value between 70 mm and 75 mm, such that the subjective refraction apparatus best matches the individual's head.

A pupillary distance scale 16 may be printed (or affixed) on the frame 10, here on the front plate 12 as shown on Figure 1, while a corresponding indicator 29 may then be printed (or affixed, or even formed) on either one of the mounts 20, 30 in a region neighbouring the pupillary distance scale 16 such that the current value of the pupillary distance may be easily grasped by the user. According to a possible variation, the pupillary distance scale may be situated on either on of the mounts 20, 30 and the indicator may then be situated on the frame 10.

As further explained below, the left lens mount 30 carries a front lens 50 and a rear lens 60 thanks to a screw 44 mounted in the sliding part 31 of the left lens mount 30. The screw 44 can be rotated by user action on a button 45 affixed to the screw 44 and located immediately to the left of the frame 10.

In a similar manner, the right lens mount 20 carries a front lens 70 and a rear lens 80 thanks to a screw 42 mounted in the sliding part 21 of the right lens mount 20. The screw 42 can be rotated by user action on a button 43 affixed to the screw 44 and located immediately to the right of the frame 10.

Herebelow is now given a detailed description of the left lens mount 30, front lens 50 and rear lens 60. The right lens mount 20, front lens 70 and rear lens 80 have a corresponding construction (deduced by symmetry along a vertical plane corresponding to a sagittal plane of the wearer of the subjective refraction apparatus) and are not therefore further described below.

The sliding part 31 of the left lens mount 30 comprises through holes 39 defining a horizontal axis perpendicular to the optical axis of the lenses 50, 60 (this optical axis corresponding to the gaze direction of wearer).

Screw 44 is mounted in the sliding part 31 through the trough holes 39 in such a manner as to be rotatable with respect to the sliding part 31 (by action on button 45 as noted above), without moving along its own (above mentioned horizontal) axis.

The front lens 50 and the rear lens 60 are each made of a transparent material, for instance a transparent plastic material such as polycarbonate.

The front lens 50 and the rear lens 60 each comprise an optical part 52, 62 (having a generally rectangular shape) and a threaded part 55, 65 extending from an upper edge of the optical part 52, 62, only in a (small) portion of this upper edge. The optical part 52, 62 and the threaded part 55, 65 are here formed integrally with each other.

In an assembled position (as visible for instance in Figure 4), the threaded part 55 of the front lens 50 is received in a first casing 37 formed in the right half of the sliding part 31 so as to be movable inside this first casing 37. The threaded part 55 has an internal thread engaging with the screw 44 such that a rotation of the screw 44 (by action on button 45) results in a translational motion of the threaded part 55 inside the first casing 37, and thus of the front lens 50.

Similarly, the threaded part 65 of the rear lens 60 is received in a second casing 38 formed in the left half of the sliding part 31 so as to be movable inside this second casing 38. The threaded part 65 has an internal thread engaging with the screw 44 such that a rotation of the screw 44 (by action on button 45) results in a translational motion of the threaded part 65 inside the second casing 38, and thus of the rear lens 60.

Internal threads of the threaded part 55 and of the threaded part 65, respectively, are constructed in opposite directions such that rotation of the screw in a given sense (e.g. clockwise) results in translational motion of the threaded part 55 in a first direction (along the above mentioned horizontal axis) and of the threaded part 65 in a second direction opposite the first direction.

The optical part 52 of the front lens 50 and the optical part 62 of the rear lens 60 extend parallel to each other and form an Alvarez lens thanks to an adapted design of their surfaces. Reference can be made for instance to the above mentioned document published as US3507565A for further details on such an Alvarez lens.

Thus, by rotating screw 44 by action on button 45, the front lens 50 and the rear lens 60 are moved in opposite directions, which makes it possible to adjust the spherical power provided (to the left eye of the individual performing the subjective refraction test) by the combination of the front lens 50 and the rear lens 60 without however moving the optical axis.

In a corresponding manner, by rotating screw 42 by action on button 43, the front lens 70 and the rear lens 80 mounted on the right lens mount 20 are moved in opposite directions, which makes it possible to adjust the spherical power provided (to the right eye of the individual) by the combination of the front lens 70 and the rear lens 80 without however moving the optical axis.

As shown in Figures 5 and 6, a spherical power scale 54 may be affixed to one of the front lens 50 and the rear lens 60 (here to the front lens 50) and a corresponding index 64 may be affixed to the other one of the front lens 50 and the rear lens 60 (here to the rear lens 60).

The spherical power scale 54 is here situated on the front face (i.e. on the side facing the lens mount 30) of the front lens 50 so as to be clearly visible by the user (e.g. the optometrist), here across the central opening 33.

In the present embodiment, the index 64 situated on the rear lens is visible (for the user) through a window 53 formed in the front lens 50. According to a possible variation, such a window could be omitted and the index 64 could be visible by transparency across the front lens 50.

In any case, the scale and index 54, 64 are visible through an aperture made in the plate 32 of the lens mount 30, here through the central aperture 33.

As the front lens 50 and the rear lens 60 form an Alvarez lens, the spherical power provided by the lens assembly (i.e. the combination of the front lens 50 and the rear lens 60) depends on the relative position of the front lens 50 and the rear lens 60 and the position of the index 64 on the spherical power scale 54 thus indicates to the user the current spherical power provided by the lens assembly.

In this respect, as the position of the index 64 on the spherical power scale 54 depends only on the relative position of the front lens 50 and the rear leas 60, the index and scale 54, 64 gives an accurate indication of the current spherical power, without needing any calibration or taking into account any additional mechanism.

Figure 5 corresponds to a situation where the threaded part 55 is to the left of the first casing 37 and the threaded part 65 is to the right of the second casing 38, as shown in Figure 4. In the present embodiment, the lens assembly 50, 60 provides a positive (non-null) spherical power in this situation, here a power of +4D.

Figure 6 corresponds to a situation where the threaded part 55 it to the right of the first casing 37 and the threaded part 65 is to the left of the second casing 38, thus providing a negative spherical power to the wearer of the subjective refraction apparatus (here a power of -4D).

The spherical power can be adjusted to other values (including a null spherical power, i.e. no spherical correction) by rotating screw 44 by action on button 45.

Figure 7 shows a possible variation for the lens mount of the subjective refraction apparatus (here a possible variation for the left lens mount 30).

According to this variation, the lens mount 130 comprises a (vertically extending) plate 132 having an aperture 133 (here a circular aperture) around the optical axis of the lenses and a window 136 facing the scale and index 54, 64 that may be provided on the lenses 50, 60 as explained above.

As in the embodiment described above, the lens mount 130 also comprises a sliding part 131 from which a teethed arm 135 extends.

The lens mount 130 further comprises a support 134, here formed by a plurality of bosses extending in a spaced relationship in the bottom region of the plate 132 and comprising a slot for receiving an additional lens, while allowing rotation of this additional lens about the optical axis (as explained above).

Markings 137 forming a cylindrical axis scale are printed on the front face of the plate 132. Thus, when mounting on the support 134 a cylindrical power lens having a mark along the axis of the cylindrical correction, the orientation of this axis of cylindrical correction can easily be grasped by the optometrist by observing where this mark is situated on the cylindrical axis scale 137.

## Claims

1. A subjective refraction apparatus providing a variable optical correction along an optical axis and comprising a pair of lenses (70, 80; 50, 60) mounted on a mount (20; 30; 130) in such a manner that the lenses (70, 80; 50, 60) are movable with respect to each other in a direction perpendicular to the optical axis for generating a variable spherical power along the optical axis,
**characterized in that** the mount (20; 30; 130) is provided with a support (24; 34; 134) defining a space for removably receiving an additional lens (6; 8) on the optical axis.

2. The subjective refraction apparatus according to claim 1, further comprising said additional lens (6; 8) mounted onto said support (24; 34; 134), wherein said additional lens (6; 8) has a cylindrical power along the optical axis.

3. The subjective refraction apparatus according to claim 1, further comprising said additional lens mounted onto said support (24; 34; 134), wherein said additional lens has a spherical power along the optical axis.

4. The subjective refraction apparatus according to any of claims 1 to 3, wherein the mount (130) includes markings (137) along a periphery of said space.

5. The subjective refraction apparatus according to any of claims 1 to 4, wherein the mount (20; 30; 130) extends perpendicularly to the optical axis and wherein said support (24; 34; 134) extends on a side of said mount (20; 30; 130) opposite another side of said mount (20; 30; 130) facing one of said movable lenses (70; 50).

6. The subjective refraction apparatus according to any of claims 1 to 5, wherein said support (24; 34; 134) extends over less than half the periphery of the space for receiving additional lens (6; 8).

7. The subjective refraction apparatus according to any of claims 1 to 6, wherein a spherical power scale (54) is affixed to a first movable lens (50) among the movable lenses.

8. The subjective refraction apparatus according to claim 7, wherein an index (64) pointing on the spherical power scale (54) is affixed to the second movable lens (60) among the movable lenses.

9. The subjective refraction apparatus according to claim 7 or 8, wherein said spherical power scale (54) extends on a side of the first movable lens (50) directed towards the space for receiving the additional lens (8).

10. The subjective refraction apparatus according to any of claims 7 to 9, wherein said mount (30; 130) defines a window (33; 136) in correspondence with said spherical power scale (54).

11. The subjective refraction apparatus according to any of claims 1 to 10, wherein each movable lens (50; 60; 70; 80) has a threaded part (55; 65) engaging a screw (44; 42) and wherein said screw (44; 42) is rotatable in the mount (30; 40) in such a manner that the movable lenses (50, 60; 70, 80) move symmetrically with respect to the mount (30; 40).

12. The subjective refraction apparatus according to claim 11, wherein said threaded part (55; 65) is a transparent part integral with an optical part (52; 62) of the concerned movable lens (50; 60).

13. The subjective refraction apparatus according to any of claims 1 to 12, wherein the mount (20; 30; 130) is mounted on a frame (10) at an adjustable position perpendicularly to the optical axis.

14. The subjective refraction apparatus according to claim 13, wherein a pinion (40) attached to a button (41) engages a rack (26; 36) affixed to the mount (20; 30).

15. The subjective refraction apparatus according to claim 13 or 14, further including a pair of temples (2, 4), wherein each temple (2; 4) is mounted to the frame (10).
